# EUROPEAN PATENT APPLICATION

(11) **EP 4 039 263 A1**
(43) Date of publication of application: **10.08.2022**
(21) Application number: 21155214.6
(22) Date of filing: 04.02.2021
(51) Int. Cl.: A61K 38/03, A61P 27/02

(54) **FORMULATION FOR THE TREATMENT OF ASTHENOPIA**

(71) Applicant: Medivis S.r.l., 95030 Tremestieri Etneo, Catania (IT)
(72) Inventor: SAITA, Maria Grazia, 95030 Tremestieri Etneo, Catania (IT); MANGIAFICO, Sergio, 95030 Tremestieri Etneo, Catania (IT); ALEO, Danilo, 95030 Tremestieri Etneo, Catania (IT); MELILLI, Barbara, 95030 Tremestieri Etneo, Catania (IT); SPITALERI, Fabiola, 95030 Tremestieri Etneo, Catania (IT); CRO, Melina, 95030 Tremestieri Etneo, Catania (IT)
(74) Representative: Croce, Valeria

(57) **Abstract**

The present patent application relates to formulations for ophthalmic application used in the treatment of asthenopia.

## Description

The present patent application describes ophthalmic formulations for the treatment of asthenopia.

### Prior art

Asthenopia, also known as eye strain, describes a series of non-specific symptoms associated with the prolonged use of video terminals, prolonged reading, or working in poorly lit environments.

Asthenopia is recognized through the onset of non-specific ophthalmic symptoms such as fatigue, pain, burning, and headache or specific symptoms such as photophobia, blurred vision, double vision, itching, tearing, and foreign body sensation.

Some studies show that asthenopic phenomena affect subjects with refractive errors, accommodation dysfunction, eye muscle problems, strabismus.

Asthenopia can be divided into internal and external asthenopia.

Internal asthenopia is caused by refractive or accommodation errors, while external asthenopia is essentially due to reading or working in poorly lit environments.

Due to the increased use of electronic devices (pc, smartphone, etc.) and other actions involving close-up work, asthenopia and associated disorders are bound to increase.

To date, the only useful actions to counter these disorders comprise the use of tear substitutes, improving workplace lighting, and reducing the hours of exposure to any electronic device.

### Summary of the invention

The authors of the present invention have surprisingly found that some peptides are capable of significantly reducing the symptoms related to eye strain (asthenopia).

### Object of the invention

In a first object, the present patent application describes compounds for medical use in the treatment of asthenopia.

In a second object, the present patent application describes an ophthalmic formulation comprising the compounds of the invention.

Ophthalmic formulations having specific compositions represent further aspects of the present invention.

### Brief description of the drawings

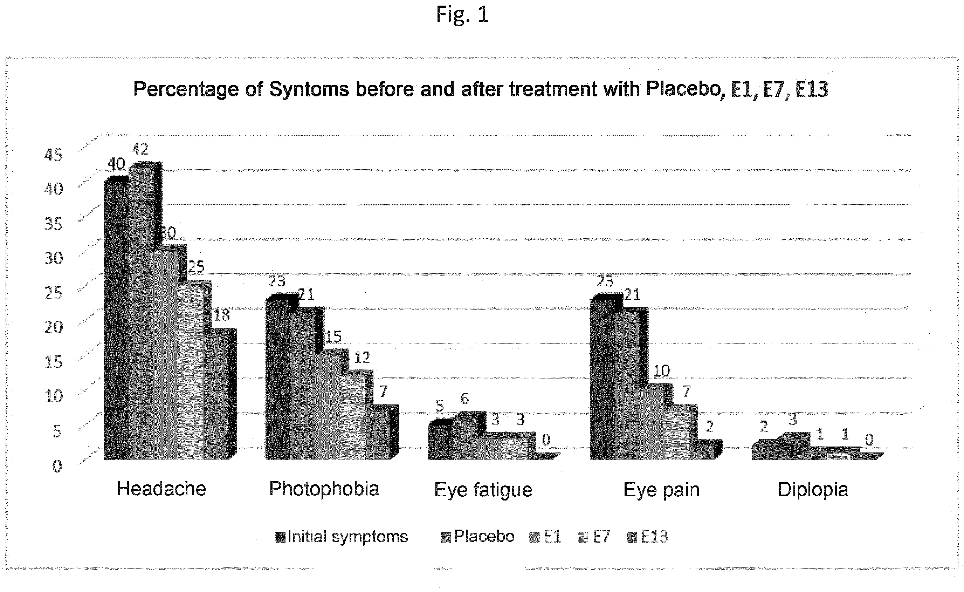

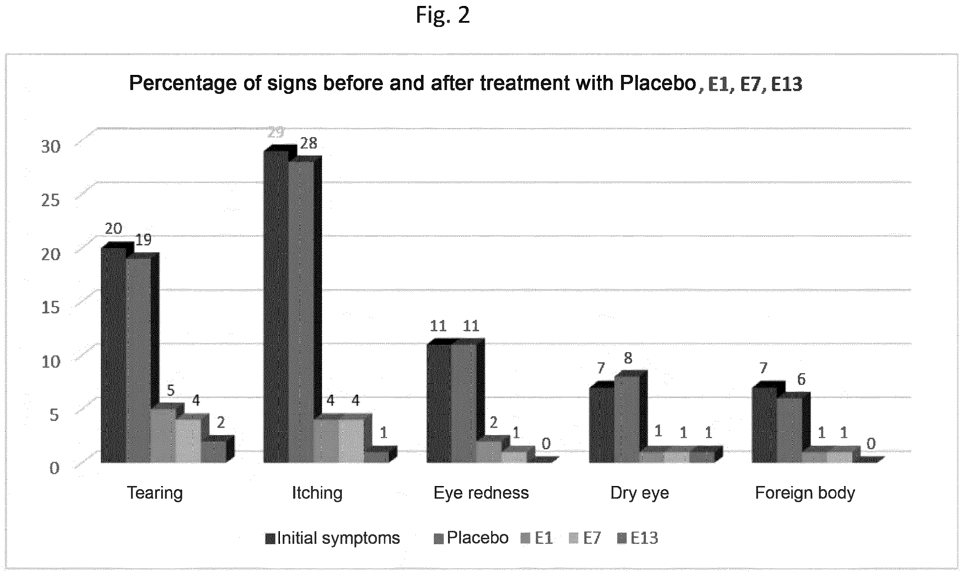

Figure 1 and figure 2 show the graphs with the results of the clinical assessments carried out using the formulations of the invention with respect to a placebo.

### Description of the invention

According to a first object, the present invention describes compounds for medical use in the treatment of asthenopia.

In particular, the term "asthenopia" refers to ocular symptoms linked to eye strain.

Such symptomatology is linked to ocular surface dysfunctions.

More in particular, such symptomatology comprises one or more of the following symptoms: headache, photophobia, eye fatigue, eye pain, diplopia, tearing, itching, red eye, dry eye, foreign body sensation.

For the purposes of the present invention, the compounds described are characterized by the following formula:
**R₁-Wₐ-X_{b}-AA₁-AA₂-AA₃-AA₄-AA₅-AA₆-Y_{c}-Z_{d}-R₂**
the stereoisomers thereof and/or the pharmaceutically acceptable salts thereof, where:
**AA₁** is Arg, Lys or Asn,
**AA₂** is Arg or Lys,
**AA₃** is Gln, Glu, Asn or Asp,
**AA₄** is Met or Leu,
**AA₅** is Glu, Asp or Gln,
**AA₆** is Glu, Asp, Gln,
**W, X, Y** and **Z** can be any amino acid independently of each other;
**a, b, c, d** can be 0 or 1 independently of each other;
**R₁** can be H, a polymeric radical of the polyethylene glycol type, a non-cyclic, cyclic aliphatic group, a heterocyclic, a heteroalkylaryl, an aryl group and R₅-CO- where R₅ can be a non-cyclic, cyclic aliphatic group, a heterocyclic, a heteroalkylaryl, an aryl group;
**R₂** can be an -NR₃R₄, -OR₃, -SR₃ group where R₃ and R₄ can be independently selected from H, a polymeric radical of the polyethylene glycol type, a non-cyclic, cyclic aliphatic group, a heterocyclic, a heteroalkylaryl, an aryl group.

In the above formula, both R₁ and R₂ are not amino acids.

In a preferred aspect, the compounds of the present invention are represented by the peptides selected from the group comprising: acetyl hexapeptide-8 (also known as acetyl hexapeptide-3), pentapeptide-3, pentapeptide-18, tripeptide-3, acetyl octapeptide-3.

Commercial examples of the above compounds are the following:

| **Compound** | **Company name** |
|---|---|
| acetyl hexapeptide-8 | Argireline^{®} |
| pentapeptide-3 | Vialox |
| pentapeptide-18 | Leuphasyl^{®} |
| tripeptide-3 | SYN-AKE^{®} |
| acetyl octapeptide-3 | SNAP-8^{®} |

In a second object, the present invention describes ophthalmic formulations comprising the compounds of the invention.

In particular, such formulations are aqueous.

For the purposes of the present invention, the described peptides are comprised, individually or in mixture, in a concentration of about 0.001-2% (weight/weight).

In preferred aspects, such compounds can be comprised in a concentration of about 0.0050-0.5 (weight/weight) or about 0.01-0.02% (weight/weight).

Furthermore, the formulations described can comprise one or more of the following further components: pH buffers or modifiers, osmotic agents, viscosifying agents, antioxidants, stabilizers.

In particular, the ophthalmic compositions of the invention can have pH values between about 5 and 8.

Citrate buffer or phosphate buffer can be used as pH buffering agents.

In one aspect, the described compositions are characterized by an osmolality of about 150 and 390 mOsm/Kg.

As osmotic agents, for example, compounds can be used selected from the group comprising: glycerol, mannitol, sorbitol, trehalose and chlorides of alkaline or alkaline-earth metals.

In order to increase tolerability, the ophthalmic compositions of the invention can contain viscosifying agents in quantities of about 0.001-4% (weight/weight).

As viscosifying agents, compounds can be used selected from the group comprising: hyaluronic acid and the salts thereof, polyacrylates such as Carbopol, cellulose such as carboxymethylcellulose (CMC) and hydroxypropyl methylcellulose (HPMC), xanthan and hydroxypropyl-guar gum (HP-G).

In one aspect, the formulations of the invention can include antioxidants and stabilizers at low concentrations.

For example, taurine can be used as an antioxidant.
Some examples of formulations as described above are reported below.

### EXAMPLE 1

### Formulations E1-E9

The following table shows examples of formulations according to the present invention having the formulations from E1 to E9.

| | **Composition % (weight/weight)** | | | | | | | | |
|---|---|---|---|---|---|---|---|---|---|
| **Ingredients** | **E1** | **E2** | **E3** | **E4** | **E5** | **E6** | **E7** | **E8** | **E9** |
| **Acetyl hexapeptide-8** | 0.0050 | | | 1.0 | | | 0.01 | | |
| **Pentapeptide-3** | | 0.0050 | | | 1.0 | | | 0.01 | |
| **Pentapeptide-18** | | | 0.0050 | | | 1.0 | | | 0.01 |
| Sodium hyaluronate | 0.10 | 0.10 | 0.10 | 0.30 | | | 0.15 | | |
| HPMC | | | | | 0.5 | | | 1.0 | |
| Carbopol | | | | | | 0.25 | | | 0.15 |
| Taurine | 0.01 | 0.01 | 0.01 | 0.01 | 0.01 | 0.01 | | 0.01 | 0.01 |
| Potassium citrate monohydrate | 0.20 | 0.20 | 0.20 | 0.20 | 0.20 | 0.20 | 0.20 | 0.20 | 0.20 |
| Citric acid monohydrate | 0.0025 | 0.0025 | 0.0025 | 0.0025 | 0.0025 | 0.0025 | 0.0025 | 0.0025 | 0.0025 |
| Glycerol | 2.2 | 2.2 | 2.2 | 2.2 | 2.2 | 2.2 | 2.2 | 2.2 | 2.2 |
| Purified water | as needed | as needed | as needed | as needed | as needed | as needed | as needed | as needed | as needed |

### EXAMPLE 2

### Formulations E10-E18

The following table shows examples of formulations according to the present invention having the formulations from E10 to E18.

| | **Composition % (weight/weight)** | | | | | | | | |
|---|---|---|---|---|---|---|---|---|---|
| **Ingredients** | **E10** | **E11** | **E12** | **E13** | **E14** | **E15** | **E16** | **E17** | **E18** |
| **Acetyl hexapeptide-8** | 0.02 | | | 2.0 | | | 0.5 | | |
| **Tripeptide-3** | | 0.0050 | | | 1.0 | | | 0.01 | |
| **Acetyl octapeptide-3** | | | 0.0050 | | | 1.0 | | | 0.01 |
| Sodium hyaluronate | 0.10 | 0.10 | 0.10 | 0.30 | | | 0.15 | | |
| HPMC | | | | | 0.5 | | | 1.0 | |
| Carbopol | | | | | | 0.25 | | | 0.15 |
| Taurine | 0.02 | 0.01 | 0.01 | | 0.01 | 0.01 | 0.01 | 0.01 | 0.01 |
| Potassium citrate monohydrate | 0.20 | 0.20 | 0.20 | 0.20 | 0.20 | 0.20 | 0.20 | 0.20 | 0.20 |
| Citric acid monohydrate | 0.0025 | 0.0025 | 0.0025 | 0.0025 | 0.0025 | 0.0025 | 0.0025 | 0.0025 | 0.0025 |
| Glycerol | 2.2 | 2.2 | 2.2 | 2.2 | 2.2 | 2.2 | 2.2 | 2.2 | 2.2 |
| Purified water | as needed | as needed | as needed | as needed | as needed | as needed | as needed | as needed | as needed |

### EXAMPLE 3

### Stability

The formulations described, stored at a temperature of 25°C±2°C/60%RH±50, after 24 months have not changed the pH and osmolality thereof beyond 10% of the initial value and the concentration of the formulated peptide has remained within 90% of the initial titer.

### EXAMPLE 4

### Clinical assessments

160 patients suffering from signs and symptoms of eye strain (asthenopia) were selected for the clinical assessments of the compositions under examination. 90 men and 70 women aged between 30 and 45 were selected, who for professional reasons used video terminals more than 6 hours a day.

The ocular symptoms were: headache, photophobia, eye pain, diplopia, tired eyes; the signs of ocular surface distress were: excessive tearing, itching, red eye, dry eye, foreign body sensation.

The frequency of symptoms and signs for the enrolled patients is shown in Table 1 below.

| **Symptom** | **%** | **Sign** | **%** |
|---|---|---|---|
| Headache | 40 | Tearing | 20 |
| Photophobia | 23 | Itching | 29 |
| Eye fatigue | 5 | Eye redness | 11 |
| Eye pain | 23 | Dry eye | 7 |
| Diplopia | 2 | Foreign object | 7 |

The 160 patients were divided into 4 groups of 40, named according to the ophthalmic composition in use: E1, E7, E13 and Placebo (PBS, phosphate buffered saline). All 160 patients enrolled were given a box of 90 single-dose vials containing the compositions in question (E1, E7, E13 and Placebo). The administration was three drops three times a day for thirty days for each eye. During the thirty days of treatment, the patients continued their normal private and professional routine and no concomitant topical or systemic treatment was performed other than the administration of the compositions E1, E7, E13 and Placebo. At the end of the trial, the signs and symptoms were assessed and these were the percentages in the three groups.

**Table 2 below shows the percentage of the signs and symptoms in the patients who administered the compositions E1, E7, E13.**

| **Symptom** | **E1** | **E7** | **E13** | **Placebo** | **Sign** | **E1** | **E7** | **E13** | **Placebo** |
|---|---|---|---|---|---|---|---|---|---|
| | % | % | % | % | | % | % | % | % |
| Headache | 30 | 25 | 18 | 42 | Tearing | 5 | 4 | 2 | 19 |
| Photophobia | 15 | 12 | 7 | 21 | Itching | 4 | 4 | 1 | 28 |
| Eye fatigue | 3 | 3 | 0 | 6 | Eye redness | 2 | 1 | 0 | 11 |
| Eye pain | 10 | 7 | 3 | 21 | Dry eye | 1 | 1 | 1 | 8 |
| Diplopia | 1 | 1 | 0 | 3 | Foreign object | 1 | 1 | 0 | 6 |

The graphs of Figures 1 and 2 show a comparison between the signs and symptoms before and after treatment with the placebo and with the formulations of the invention E1, E7 and E13.

As demonstrated by the results shown in graphs 1-2, the formulations E1, E7 and E13, i.e., those containing the peptides of the invention, surprisingly reduced the signs and symptoms related to eye strain.

It can therefore be concluded that such peptides have surprisingly shown that they can be used effectively in the improvement of clinical parameters related to eye strain in the treatment of asthenopia.

## Claims

1. A peptide having the formula
**R₁-Wₐ-X_{b}-AA₁-AA₂-AA₃-AA₄-AA₅-AA₆-Y_{c}-Z_{d}-R₂** the stereoisomers thereof and/or the pharmaceutically acceptable salts thereof, wherein:
**AA₁** is Arg, Lys or Asn,
**AA₂** is Arg or Lys,
**AA₃** is Gln, Glu, Asn or Asp,
**AA₄** is Met or Leu,
**AA₅** is Glu, Asp or Gln,
**AA₆** is Glu, Asp, Gln,
**W, X, Y** and **Z** can be any amino acid independently of each other;
**a, b, c, d** can be 0 or 1 independently of each other;
**R₁** can be H, a polymeric radical of the polyethylene glycol type, a non-cyclic, cyclic aliphatic group, a heterocyclic, a heteroalkylaryl, an aryl group and R₅-CO- where R₅ can be a non-cyclic, cyclic aliphatic group, a heterocyclic, a heteroalkylaryl, an aryl group;
**R₂** can be an -NR₃R₄, -OR₃, -SR₃ group where R₃ and R₄ can be independently selected from H, a polymeric radical of the polyethylene glycol type, a non-cyclic, cyclic aliphatic group, a heterocyclic, a heteroalkylaryl, an aryl group, and wherein R₁ and R₂ are not amino acids,
for medical use in the treatment of asthenopia.

2. A peptide according to the preceding claim for medical use in the treatment of asthenopia, which is selected from the group comprising: acetyl hexapeptide-8, pentapeptide-3, pentapeptide-18, tripeptide-3, acetyl octapeptide-3.

3. An ophthalmic formulation comprising one or a mixture of peptides according to claim 1 or 2.

4. An ophthalmic formulation according to the preceding claim, wherein said peptide or said peptide mixture is comprised in a concentration of about 0.001-2% (weight/weight).

5. An ophthalmic formulation according to the preceding claim 3 or 4, wherein said peptide or said peptide mixture is comprised in a concentration of about 0.0050-0.5 (weight/weight) or about 0.01-0.02% (weight/weight).

6. An ophthalmic formulation according to any one of the preceding claims 3 to 5, having osmolality of about 150 and 390 mOsm/Kg.

7. An ophthalmic formulation according to any one of the preceding claims 3 to 6, further comprising one or more of the following agents: pH buffers or modifiers, viscosifying agents, antioxidants, stabilizers.

8. An ophthalmic formulation according to any one of the preceding claims 3 to 7, wherein said viscosifying agents are comprised in an amount of about 0.001-4% (weight/weight).

9. An ophthalmic formulation according to any one of the preceding claims 3 to 8, having one of the following compositions:
| | **Composition % (weight/weight)** | | | | | | | | |
|---|---|---|---|---|---|---|---|---|---|
| **Ingredients** | **E1** | **E2** | **E3** | **E4** | **E5** | **E6** | **E7** | **E8** | **E9** |
| **Acetyl hexapeptide-8** | 0.0050 | | | 1.0 | | | 0.01 | | |
| **Pentapeptide-3** | | 0.0050 | | | 1.0 | | | 0.01 | |
| **Pentapeptide-18** | | | 0.0050 | | | 1.0 | | | 0.01 |
| Sodium hyaluronate | 0.10 | 0.10 | 0.10 | 0.30 | | | 0.15 | | |
| HPMC | | | | | 0.5 | | | 1.0 | |
| Carbopol | | | | | | 0.25 | | | 0.15 |
| Taurine | 0.01 | 0.01 | 0.01 | 0.01 | 0.01 | 0.01 | | 0.01 | 0.01 |
| Potassium citrate monohydrate | 0.20 | 0.20 | 0.20 | 0.20 | 0.20 | 0.20 | 0.20 | 0.20 | 0.20 |
| Citric acid monohydrate | 0.0025 | 0.0025 | 0.0025 | 0.0025 | 0.0025 | 0.0025 | 0.0025 | 0.0025 | 0.0025 |
| Glycerol | 2.2 | 2.2 | 2.2 | 2.2 | 2.2 | 2.2 | 2.2 | 2.2 | 2.2 |
| Purified water | as needed | as needed | as needed | as needed | as needed | as needed | as needed | as needed | as needed |
or
| | **Composition % (weight/weight)** | | | | | | | | |
|---|---|---|---|---|---|---|---|---|---|
| **Ingredients** | **E10** | **E11** | **E12** | **E13** | **E14** | **E15** | **E16** | **E17** | **E18** |
| **Acetyl hexapeptide-8** | 0.02 | | | 2.0 | | | 0.5 | | |
| **Tripeptide-3** | | 0.0050 | | | 1.0 | | | 0.01 | |
| **Acetyl octapeptide-3** | | | 0.0050 | | | 1.0 | | | 0.01 |
| Sodium hyaluronate | 0.10 | 0.10 | 0.10 | 0.30 | | | 0.15 | | |
| HPMC | | | | | 0.5 | | | 1.0 | |
| Carbopol | | | | | | 0.25 | | | 0.15 |
| Taurine | 0.02 | 0.01 | 0.01 | | 0.01 | 0.01 | 0.01 | 0.01 | 0.01 |
| Potassium citrate monohydrate | 0.20 | 0.20 | 0.20 | 0.20 | 0.20 | 0.20 | 0.20 | 0.20 | 0.20 |
| Citric acid monohydrate | 0.0025 | 0.0025 | 0.0025 | 0.0025 | 0.0025 | 0.0025 | 0.0025 | 0.0025 | 0.0025 |
| Glycerol | 2.2 | 2.2 | 2.2 | 2.2 | 2.2 | 2.2 | 2.2 | 2.2 | 2.2 |
| Purified water | as needed | as needed | as needed | as needed | as needed | as needed | as needed | as needed | as needed |
